# EUROPEAN PATENT APPLICATION

(11) **EP 2 158 912 A1**
(43) Date of publication of application: **03.03.2010**
(21) Application number: 08014983.4
(22) Date of filing: 25.08.2008
(51) Int. Cl.: A61K 31/517

(54) **Pharmaceutical composition comprising N-[3-chhloro-4-[3-fluorophenyl)methoxy)phenyl]6-[5[[[2-(methylsulfonyl)ethyl]amino]methyl]-2-furyl]-4-quinazolinamine**

(71) Applicant: Ratiopharm GmbH, 89079 Ulm (DE)
(72) Inventor: Rimkus, Katrin, 80798 Munich (DE); Muskulus, Frank, 82194 Gröbenzell (DE); Brueck, Sandra, 85570 Ottenhofen (DE); Paetz, Jana, 86424 Dinkelscherben (DE)
(74) Representative: Teipel, Stephan

(57) **Abstract**

The present invention relates to a pharmaceutical composition comprising N-[3-chloro-4-[(3-fluorophenyl)methoxy]phenyl]-6-[5[[[2-(methylsulfonyl)ethyl]amino]methyl]-2-furyl]-4-quinazolinamine as active pharmaceutical ingredient and a process of preparing such composition.

## Description

### Field of the invention

The present invention relates to a pharmaceutical composition comprising N-[3-chloro-4-[(3-fluorophenyl)methoxy]phenyl]-6-[5[[[2-(methylsulfonyl)ethyl]amino]methyl]-2-furyl]-4-quinazolinamine as active pharmaceutical ingredient and a process of preparing such composition.

### Background of the invention

The compound N-[3-chloro-4-[(3-fluorophenyl)methoxy]phenyl]-6-[5[[[2-(methylsulfonyl) ethyl]amino]methyl]-2-furyl]-4-quinazolinamine (INN: Lapatinib) is a tyrosine kinase inhibitor which dually inhibits the growth factor receptors ErbB1 (epidermal growth factor receptor, EGFR) and ErbB2 (HER2). Lapatinib is a member of the 4-anilinoquinazoline class of kinase inhibitors that have been shown to bind to the ATP binding site of protein kinases and compete with the ATP substrate. This blocks receptor phosphorylation and activation, preventing subsequent downstream signalling events.

Lapatinib, in combination with Capecitabine, is indicated for the treatment of patients with advanced or metastatic breast cancer whose tumours overexpress ErbB2 (Her2) and who have received prior therapy including trastuzumab.

Lapatinib and its pharmaceutical effects on disorders like cancer are described in WO 1999/035146. Further medical uses of Lapatinib and its salts are inter alia known from WO 2005/120504, WO 2006/002422 and WO 2006/066267.

WO 1999/035146 discloses a process of preparing Lapatinib. According to this and other known manufacturing processes, Lapatinib is obtained as a solid. One of the polymorphic forms of Lapatinib is the crystalline ditosylate salt as described in WO 2002/002552.

Conventional pharmaceutical Lapatinib formulations for oral administration are disclosed in WO 2006/113649. According to the general description of this document the active ingredient can be present in a broad range of 5 to 85 % by weight. However, formulations of the examples contain only 45 % by weight of the active ingredient.

Typically, Lapatinib is administered at a dose of 1250 mg once daily. Tablets comprising 250 mg Lapatinib (as ditosylate salt monohydrate) are sold under the brand name Tyverb® (by Glaxo Smith Kline). Thus, the required dosage is comprised in 5 Tyverb® tablets that have to be administered perorally once a day. This situation is unsatisfactory and inconvenient to the patient especially since cancer patients' medications usually consist of multiple drug regimen demanding the administration of large numbers of tablets or capsules often along with an intravenous therapy. Further, these patients often suffer from nausea and lesions of the oral mucosa. Therefore the peroral application of drugs may be hampered by vomiting fits and swallowing problems. Hence, it would be desirable to reduce the number of tablets to be swallowed, thereby facilitating the administration of the cancer patients' daily medication.

Moreover, good homogeneity, flow and cohesive characteristics, stability, and (if applicable) compressibility of a pharmaceutical composition are prerequisites for a successful manufacture of for example tablets on a production scale. Further, the resulting composition should possess sufficient dissolution properties. Exactly these prerequisites are often difficult to achieve when working with high concentrations of active pharmaceutical ingredients as shown by the fact that e.g. Tyverb® tablets contain only 250 mg of Lapatinib corresponding to a drug load of less than 50 wt.% referring to the total weight of the tablet.

It is therefore an object of the invention to provide a pharmaceutical composition comprising enough active pharmaceutical ingredient to reduce the number of tablets to be taken and at the same time warranting the technical necessities described above.

It has now surprisingly been found that the above problems can be overcome by providing a pharmaceutical composition comprising a high percentage of Lapatinib.

Thus, the present invention relates to a pharmaceutical composition comprising N-[3-chloro-4-[(3-fluorophenyl)methoxy]phenyl]-6-[5[[[2-(methylsulfonyl)ethyl]amino]methyl]-2-furyl]-4-quinazolinamine or a pharmaceutically acceptable salt thereof as active pharmaceutical ingredient, wherein the active pharmaceutical ingredient is present in an amount of more than 60 wt.%, preferably 61 to 90 wt.% based on the total weight of the composition.

### Description of the invention

N-[3-chloro-4-[(3-fluorophenyl)methoxy]phenyl]-6-[5[[[2-(methylsulfonyl)ethyl]amino]methyl]-2-furyl]-4-quinazolinamine (INN: Lapatinib) has the following chemical structure:

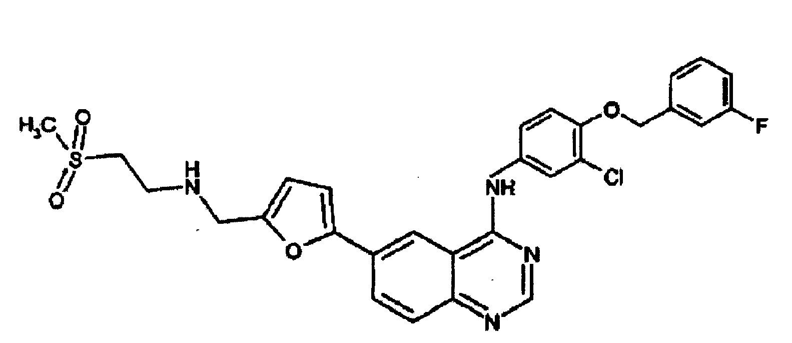

Lapatinib can be readily synthesized using techniques well known in the art. A synthesis of Lapatinib is disclosed for example in WO 1999/035146.

Ditosylate salt forms of Lapatinib as well as processes for their manufacture are disclosed in WO 2002/002552.

The term "active pharmaceutical ingredient" (API) refers to Lapatinib and to pharmaceutically acceptable salts thereof. The API can be present in any polymorphic form, including solvates and hydrates. Lapatinib ditosylate monohydrate is especially preferred.

The term "pharmaceutical composition" refers to single dosage forms, such as tablets, capsules, pellets, etc., as well as powders or granules which are used in the preparation of single dosage forms. Where it is referred to the total weight of the pharmaceutical composition and the pharmaceutical composition in a single dosage form the total weight is the weight of the single dosage form excluding, if applicable, the weight of any coating or capsule shell. The preferred dosage form of the invention is a film coated tablet.

The active pharmaceutical ingredient is present in the pharmaceutical composition in an amount of more than 60 wt.%, preferably 61 to 90 wt.%, more preferably 70 to 90 wt.%, even more preferred 80 to 90 wt.%, and most preferred more than 85 to 90 wt.% based on the total weight of the composition. Alternatively, the active pharmaceutical ingredient is present in the pharmaceutical composition in an amount of 61 to 80 wt.%, preferably 61 to 70 wt.%, and most preferred 61 to 65 wt.% based on the total weight of the composition.

Advantageous properties with respect to solubility, homogeneity, stability, flowability, compressibility and the avoidance of demixing tendencies are achieved if the active pharmaceutical ingredient used in the preparation of the pharmaceutical composition of the present invention has a mean particle size of 1 to 30 µm, preferably 1 to 20 µm, more preferably 1 to 15 µm. In one embodiment the active pharmaceutical ingredient has a specific surface area of 5 to 10 m²/g. The latter is measured according to the gas adsorption method (BET method), and the particle size distribution is determined via laser scattering performed on the API dispersed in a suspending medium. The above mentioned particle size range and/or the above specific surface area range are advantageous also with respect of a fast dissolution of a pharmaceutical composition containing a high drug load of more than 60 wt.%.

Since the active pharmaceutical ingredient as obtained from the manufacturing process may vary in its particle size, it might have to be milled or ground in order to obtain the desired mean particle size. The inventors have encountered problems with the grinding of Lapatinib ditosylate monohydrate due to its long needle shape. It was found that these problems can be overcome for example by adding one or more excipients to the milling or grinding procedure.

The grinding can for example be performed using pin or hammer mills. By varying the rotation speed of the mill, the influent of the API and/or the duration of milling, particles possessing the desired particle size and/or specific surface area can be obtained.

The use of an air jet mill is advantageous if a specific surface area of e.g. more than 6 m²/g is desired.

The API particle size and/or specific surface area can also be adjusted by mixing APIs of different particle sizes and/or specific surface areas and/or screening with sieves of suitable mesh sizes.

A bulk density of the pharmaceutical composition ranging from of 0.3 to 0.9 g/ml, preferably of 0.4 to 0.8 g/m is advantageous.

The pharmaceutical composition of the invention preferably has a Hausner ratio in the range of 1.05 to 1.65, more preferably of 1.1 to 1.5. The Hausner ratio is the ratio of bulk density to tapped density.

The pharmaceutical composition of the present invention may further comprise one or more pharmaceutically acceptable excipients, such as fillers, binding agents, lubricants, flow enhancers, antisticking agents, disintegrating agents and solubilizers. As pharmaceutically acceptable excipients conventional excipients known to the person skilled in the art may be used. See for example "Lexikon der Hilfsstoffe für Pharmazie, Kosmetik und angrenzende Gebiete", edited by H. P. Fiedler, 4th Edition, Edito Cantor, Aulendorf and earlier editions, and "Handbook of Pharmaceutical Excipients", Third Edition, edited by Arthur H. Kibbe, American Pharmaceutical Association, Washington, USA, and Pharmaceutical Press, London.

Preferred examples of the fillers are lactose, mannitol, sorbitol or microcrystalline cellulose. The filler is suitably present in an amount of 0 to 30 wt.% of the total weight of the composition.

The binding agent can for example be microcrystalline cellulose (MCC) or hydroxypropylmethyl cellulose (HPMC). Preferably, the binding agent is present in an amount of 1 to 25 wt.%, more preferably at 2 to 10 wt.% of the total weight of the composition.

The lubricant is preferably a stearate such as earth alkali metal stearate, such as magnesium stearate or sodium stearyl fumarate. The lubricant is suitably present in an amount of 0.5 to 2.5 wt.% of the total weight of the composition.

Suitable disintegrating agents are for example cellulose derivatives such croscarmellose sodium, starch derivatives such as sodium carboxymethyl starch, pregelatinized starch, synthetic polymers such as cross-linked polyvinylpyrrolidone (crospovidone) or sodium bicarbonate. The disintegrant or disintegrant mixture is suitably present in an amount of 1 to 20 wt.%, more preferably at about 5 to 10 wt.% of the total weight of the composition.

The flow enhancer can for example be colloidal silicon dioxide. Preferably, the flow enhancer is present in an amount of 0.5 to 8 wt.%, more preferably at 1 to 4 wt.% of the total weight of the composition.

The antisticking agent is for example talcum and may be present in amounts of 1 to 5 %.wt, more preferably in an amount of 1.5 to 3 wt.% of the total weight of the composition.

An improvement of the solubility of the active pharmaceutical ingredient can for example be achieved by the addition of complex forming agents/compounds (e.g. sodium benzoate, sodium salicylate or cyclodextrins), the alternation of solvent properties (e.g. by adding PVP or polyethylene glycols) or the addition of solubilizers which form tenside micelles (e.g. surfactants).

Suitable solubilizers are for example surfactants such as polyoxyethylene alcohol ethers (e.g. Brij®), polysorbates (e.g. Tween®) or polyoxypropylene polyoxyethylene copolymers (poloxamer; e.g. Pluronic®) and may be present in amounts of 0.5 to 7 wt.%, more preferably 1 to 5 wt.% of the total weight of the composition.

Alternatively, a pseudo-emulsifier can be used. Its mechanism of action mainly relies on an enhancement of viscosity. However, pseudo-emulsifiers also possess emulsifying properties.

Preferred pseudo-emulsifiers are for example cellulose ethers, gum Arabic or tragacanth. Pseudo-emulsifiers may be present in amounts of 1 to 10 wt.%, more preferably 3 to 7 wt.% of the total weight of the composition.

A person skilled in the art may use these or other excipients depending on the selected process of preparing the pharmaceutical composition of the invention.

The pharmaceutical composition of the present invention can be formulated in any known manner, preferably as tablets, capsules, granules, pellets or sachets. A particularly preferred pharmaceutical composition is in the form of tablets. The pharmaceutical composition may contain for example dosage amounts of 250, 500, 625 or 1250 mg of Lapatinib. Thus the administered amount can be readily varied according to individual tolerance and safety.

The pharmaceutical composition of the present invention can be manufactured according to standard methods known in the art. Granulates according to the invention can be obtained by dry compaction or wet granulation. These granulates can subsequently be mixed with e.g. suitable disintegrating agents, glidants and lubricants and the mixture can be compressed into tablets or filled into sachets or capsules of suitable size.

Tablets can also be obtained by direct compression of a suitable powder mixture, i.e. without any preceding granulation of the excipients.

Suitable powder or granulate mixtures according to the invention are also obtainable by spray drying, lyophilization, melt extrusion, pellet layering, coating of the active pharmaceutical ingredient or any other suitable method. Preferably, the conditions are chosen such as to prevent amorphization of the active pharmaceutical ingredient. The so obtained powders or granulates can be mixed with one or more suitable ingredients and the resulting mixtures can either be compressed to form tablets or filled into sachets or capsules.

The above mentioned methods known in the art also include grinding and sieving techniques permitting the adjustment of desired particle size distributions.

### Figures

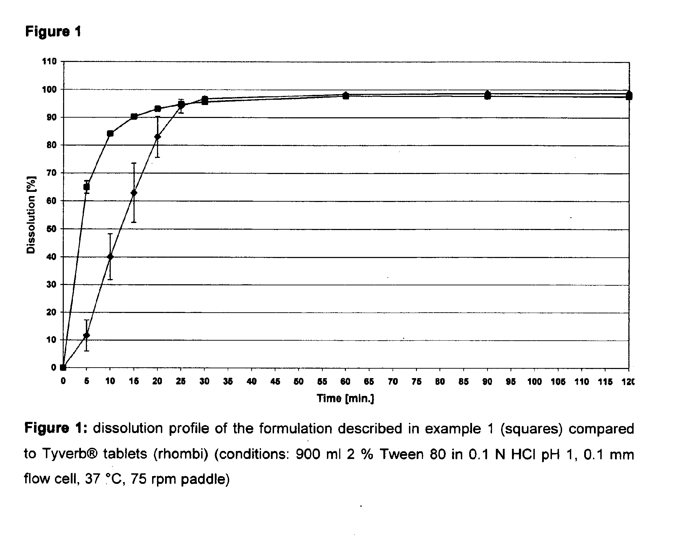

Figure 1 shows the dissolution profile of the tablet obtained in example 1.

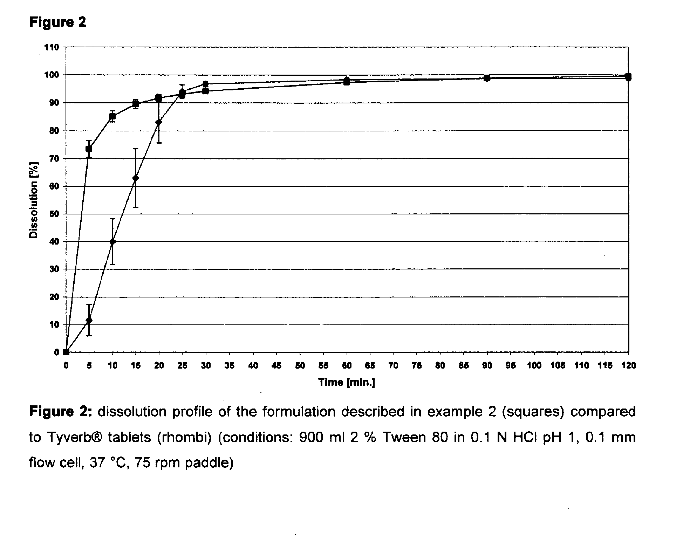

Figure 2 shows dissolution profile of the tablet obtained in example 2.

### Examples

The invention is now illustrated in the following examples which are not to be constructed as being limiting.

### Example 1

| **ingredient** | **product, e.g.** | **amount [mg]** |
|---|---|---|
| Lapatinib ditosylate monohydrate | | 406 |
| corn starch | | 180 |
| croscarmelose sodium | Ac-Di-Sol® | 30 |
| colloidal silicon dioxide | Aerosil® 200 | 20 |
| HPMC | Pharmacoat® 603 | 20 |
| sodium stearyl fumarate | Pruv® | 10 |

Lapatinib ditosylate monohydrate, corn starch and colloidal silicon dioxide are mixed and sieved, and then wet granulated with a solution of 10 % HPMC in purified water. The granulate is dried and then successively mixed with croscarmellose sodium and sodium stearyl fumarate. The mixture is compressed into tablets.

The dissolution of these tablets is faster than that of the reference (Tyverb® tablets). After 5, 10, 15 and 20 minutes 73.5 %, 84.2 %, 90.3 % and 93.1 % of the active pharmaceutical ingredient are dissolved from the formulation, compared to only 11.6 %, 40 %, 63 % and 83 % from the reference product.

### Example 2

| **ingredient** | **product, e.g.** | **amount [mg]** |
|---|---|---|
| Lapatinib ditosylate monohydrate | | 406 |
| sodium bicarbonate | | 20 |
| crosslinked PVP | Kollidon® CL | 15 |
| HPMC | Pharmacoat® 603 | 10 |
| sodium stearyl fumarate | Pruv® | 10 |
| colloidal silicon dioxide | Aerosil® 200 | 12.5 |

Lapatinib ditosylate monohydrate, sodium bicarbonate and 80 % of the colloidal silicon dioxide are mixed and sieved, and then wet granulated with a solution of HPMC in purified water. The dried granulate is mixed with sodium stearyl fumarate and the residual colloidal silicon dioxide. The mixture is compressed into tablets.

The dissolution of these tablets is faster than that of the reference (Tyverb® tablets). After 5, 10, 15 and 20 minutes 73.5 %, 85.2 %, 89.5 % and 91.7 % of the active pharmaceutical ingredient are dissolved from the formulation, compared to only 11.6 %, 40 %, 63 % and 83 % from the reference product.

## Claims

1. Pharmaceutical composition comprising N-[3-chloro-4-[(3-fluorophenyl)methoxy]phenyl]-6-[5[[[2-(methylsulfonyl)ethyl]amino]methyl]-2-furyl]-4-quinazolinamine or a pharmaceutically acceptable salt thereof as active pharmaceutical ingredient, wherein the active pharmaceutical ingredient is present in an amount of more than 60 % by weight based on the total weight of the composition.

2. The pharmaceutical composition according to claim 1, wherein the active pharmaceutical ingredient is the ditosylate salt of N-[3-chloro-4-[(3-fluorophenyl)methoxy]phenyl]-6-[5[[[2-(methylsulfonyl)ethyl]amino]methyl]-2-furyl]-4-quinazolinamine, preferably in its monohydrate form.

3. The pharmaceutical composition according to any of the preceding claims, wherein the active pharmaceutical ingredient has a mean particle size of 1 to 30 µm, preferably 1 to 20 µm.

4. The pharmaceutical composition according to any of the preceding claims, wherein the active pharmaceutical ingredient has a specific surface area of 5 to 10 m²/g.

5. The pharmaceutical composition according to any of the preceding claims having a bulk density of 0.3 to 0.9 g/ml, preferably 0.4 to 0.8 g/ml.

6. The pharmaceutical composition according to any of the preceding claims having a Hausner ratio of 1.05 to 1.65, preferably 1.1 to 1.5.

7. Process of preparing a pharmaceutical composition according to any of claims 1-6 comprising the step of milling or grinding the active pharmaceutical ingredient in the presence of one or more excipients.
